# EUROPÄISCHE PATENTSCHRIFT

(11) **EP 2 387 396 B1**
(45) Veröffentlichungstag und Bekanntmachung des Hinweises auf die Patenterteilung: **15.11.2017**
(21) Anmeldenummer: 10702639.5
(22) Anmeldetag: 14.01.2010
(51) Int. Cl.: A61K 31/15, A61K 31/17, A61K 31/195, A61K 31/198, A61K 31/405, A61K 31/42, A61K 31/422, A61K 31/52, A61K 31/522, A61K 31/7008, A61K 31/7072, A61K 45/06, A61P 17/00, A61P 31/10

(54) **MITTEL ZUR VORBEUGUNG UND BEHANDLUNG DER PITYRIASIS VERSICOLOR**
AGENT FOR PREVENTING AND TREATING PITYRIASIS VERSICOLOR
AGENT POUR LA PRÉVENTION ET LE TRAITEMENT DU PITYRIASIS VERSICOLOR

(30) Priorität: 14.01.2009 DE 102009004959; 29.07.2009 DE 102009035114
(43) Veröffentlichungstag der Anmeldung: 23.11.2011
(73) Patentinhaber: Justus-Liebig-Universität Gießen, 35390 Gießen (DE)
(72) Erfinder: MAYSER, Peter, 35444 Biebertal (DE)
(74) Vertreter: Patentanwälte Olbricht Buchhold Keulertz
(86) Internationale Anmeldenummer: PCT/EP2010/050397
(87) Internationale Veröffentlichungsnummer: WO 2010/081849

(56) Entgegenhaltungen:
- WO-A2-2007/054833
- US-A1- 2008 138 417
- MAYSER PETER ET AL: "Rapid reversal of hyperpigmentation in pityriasis versicolor upon short-term topical cycloserine application" MYCOSES, Bd. 52, Nr. 6, November 2009 (2009-11), Seiten 541-543, XP002574531 ISSN: 0933-7407
- DATABASE BIOSIS [Online] BIOSCIENCES INFORMATION SERVICE, PHILADELPHIA, PA, US; 1975, KORSUN V F ET AL: "SOME PROBLEMS OF THE PATHOGENESIS AND THERAPY OF TINEA VERSICOLOR" XP002574532 Database accession no. PREV197662049372 -& KORSUN V F ET AL: "SOME PROBLEMS OF THE PATHOGENESIS AND THERAPY OF TINEA VERSICOLOR" VESCI AKADEMII NAVUK BSSR. SERYA BIALAGICNYH NAVUK - IZVESTIAAKADEMII NAUK BSSR. SERIA BIOLOGICESKIH NAUK, NAVUKA I TEHNIKA, MINSK, BY, Nr. 4, 1. Januar 1975 (1975-01-01), Seiten 104-106, XP008120437 ISSN: 0002-3558
- DATABASE WPI Week 200873 Thomson Scientific, London, GB; AN 2008-M35576 XP002574533 -& CN 101 229 234 A (LI S) 30. Juli 2008 (2008-07-30)
- ZUTHER KATJA ET AL: "The tryptophan aminotransferase Tam1 catalyses the single biosynthetic step for tryptophan-dependent pigment synthesis in Ustilago maydis" MOLECULAR MICROBIOLOGY, Bd. 68, Nr. 1, April 2008 (2008-04), Seiten 152-172, XP002574534 ISSN: 0950-382X
- THOMA W ET AL: "Pityriasis versicolor alba." JOURNAL OF THE EUROPEAN ACADEMY OF DERMATOLOGY AND VENEREOLOGY : JEADV MAR 2005, Bd. 19, Nr. 2, März 2005 (2005-03), Seiten 147-152, XP002574535 ISSN: 0926-9959

## Beschreibung

Die vorliegende Erfindung betrifft die Verwendung von mindestens einem Transaminaseinhibitor, nämlich von Aminooxyacetat, Cycloserin oder des Derivates von Cycloserin, nämlich Terizidon, zur Herstellung eines Mittels zur Vorbeugung und Behandlung der Kleienflechte (Pityriasis versicolor).

### Beschreibung des allgemeinen Gebietes der Erfindung

Die Kleienflechte (Pityriasis versicolor) ist eine der häufigsten Pilzerkrankungen des Menschen. Sie wird durch Hefepilze der Gattung Malassezia (anderer Name: Pityrosporum ovale, orbiculare), insbesondere auch durch Malassezia furfur hervorgerufen. Malassezia-Hefen gehören zur normalen Hautflora des Menschen und vieler Warmblüter. Bei entsprechender Veranlagung, oft zusammen mit starkem Schwitzen, kann sich der Pilz aber vermehren, Pilzfäden (Hyphen) ausbilden und die Kleienflechte verursachen. Sie ist durch schuppende hyper- oder hypopigmentierte Läsionen gekennzeichnet, die trotz hoher Pilzlast keine oder nur eine geringe entzündliche Aktivität aufweisen. Die Läsionen zeigen eine charakteristische gelb-grünliche Fluoreszenz, die diagnostisch genutzt wird. Die Erkrankung zeichnet sich durch hell- bis dunkelbraune, z. T. erythematöse Maculae aus, die bevorzugt in den talgdrüsenreichen Arealen des Körperstammes zu finden sind. Die Herde sind zunächst linsen- bis pfenniggroß (ca. 1,5 cm) und neigen zu landkartenartiger Konfluenz. Beim Streichen über die Läsionen mit einem Holzspatel fällt eine feine, kleieartige (= pityriasiforme) Schuppung ("Hobelspanphänomen") auf. Häufig unter UV-Bestrahlung, aber auch an bedeckten Körperstellen kann es zu einer Umwandlung ("versicolor") der hyperpigmentierten Areale in weiße, nicht oder nur gering schuppende Läsionen kommen (Pityriasis versicolor alba). Die Repigmentierung kann bis zu einigen Monaten dauern.

Abgesehen von diesen oft sehr störenden kosmetischen Aspekten sind die Patienten nicht beeinträchtigt, gelegentlich wird leichter Juckreiz beschrieben, vor allem bei stärkerem Schwitzen. Betroffen sind vor allem Jugendliche und jüngere Menschen in der 2. und 3. Lebensdekade, nach dem 60. Lebensjahr ist die Inzidenz deutlich geringer. Mit Ausnahme der Tropen findet sich die Erkrankung selten bei unter 10-jährigen, da die Veränderung der Hautlipide in der Pubertät bedeutsam ist. Ausgesprochene Geschlechtspräferenz besteht nicht. Deutlich ist der Einfluss des Makroklimas. In tropischen bis subtropischen Regionen ist etwa jeder Zweite erkrankt. In Nord- und Mitteleuropa hat Pityriasis versicolor eine Inzidenz von 0,5 - 1 % mit einem Maximum in den Monaten Mai bis September.

Die Kontagiosität gilt als gering oder nicht gegeben. Epidemisches Auftreten bzw. Partnerinfektionen wurden nur selten beschrieben. Experimentell ließ sich die Erkrankung jedoch in Einzelfällen reproduzieren. Zu den Prädispositionsfaktoren zählen, neben tropisch-feuchtwarmem Makroklima, auch individuelle Schweißneigung (z. B. bei Hyperthyreose, Tuberkulose, Malignomen) und Einflüsse des Mikroklimas (Okklusion), ferner Malnutrition, eine positive Familienanamnese und die Anwendung lipidhaltiger Externa. Gegen eine maßgebliche pathogenetische Bedeutung einer Immunsuppression spricht die unveränderte Inzidenz bei gleichzeitiger Erkrankung an Diabetes mellitus oder AIDS.

Die Diagnosestellung "Pityriasis versicolor" erfolgt meist klinisch, ergänzt durch eine Wood-Licht-Untersuchung (gelblich-grüne Fluoreszenz) und das KOH-Nativpräparat. Mikroskopisch finden sich in den Schuppen sehr charakteristisch kurze, z. T. fragmentierte Pilzfäden neben runden Hefezellen ("Spaghetti und Fleischklößchen"). Auf das Anlegen einer Pilzkultur (lipidhaltige Medien wie Dixon- oder Leeming-Notman-Agar) kann verzichtet werden, da der Erreger zur residenten Keimflora der menschlichen Haut gehört. *M. globosa* wurde jedoch in den Läsionen als dominierende Spezies bei Pityriasis versicolor nachgewiesen und aufgrund der runden Sprosszellen als pathogenes Agens vermutet.

### Stand der Technik

Die Krankheit ist behandelbar, neigt aber stark zu rezidivierendem Verlauf. Zu den Allgemeinmaßnahmen zählen häufiges Baden/Duschen unter Verwendung von Syndets, Vermeidung von Kleidung mit Okklusiveffekt und Ausschalten von Prädispositionsfaktoren. Topisch wirksam sind Antimykotika aus der Gruppe der Azole, Ciclopiroxolamin und Terbinafin, daneben aber auch klassische Therapeutika wie Natriumthiosulfat, Pyrithion-Zink, Schwefel, Propylenglykol und Selendisulfid. Topisch sollte immer der gesamte Körper behandelt werden, wirkstoffhaltige Shampoos oder Lösungen werden daher bevorzugt. Die systemische Therapie ist bei ausgedehnten Herden und häufigen Rezidiven indiziert. Zur Verfügung stehen die Azole Itraconazol und Fluconazol.

Leider kann die Erkrankung durch die bisherige Behandlung zwar vorübergehend zum Verschwinden gebracht, aber nicht geheilt werden, da immer einige Hefepilze auf der Haut zurückbleiben, die dann für eine neue Erkrankung verantwortlich sein können. Zudem sind insbesondere die depigmentierten Hautareale, einmal entstanden, auch der Behandlung mit Antipilzmitteln nicht mehr zugänglich.
Der Stand der Technik kennt zur Behandlung der Kleienflechte diverse Shampoos, Salben, Gele und Sprays, die Antimykotika (z.B. Ketoconazol, Terbinafin) oder Seleniumsulfid beinhalten. Obwohl die Erkrankung mit diesen Antimykotika behandelt wird, zeigt sie eine sehr hohe Rezidivneigung und störende Folgeerscheinungen bei verspäteter Therapie. Die Mittel erfordern eine regelmäßige, tägliche Behandlung, jedoch können sie das Wiedererscheinen des Pilzes und der Symptome nicht verhindern. Oft bleibt die assoziierte Hypopigmentierung noch für Monate bestehen. Als homöopathische Mittel werden Kaliumchloratum und Kaliumphosphoricum vorgeschlagen.

Die WO 2007/054833 A2 zeigt die Verwendung von Hydroxyalkylharnstoffen in einer kosmetischen Zubereitung zur Behandlung von Hauterkrankungen. Dabei wird allerdings auf die antimykotische Wirkung von Hydroxyalkylharnstoffen, inbesondere von Hydroxyethylharnstoff, auf Hefezellen, wie beispielsweise der Gattung wie Melassezia, abgestellt. Die Verwendung eines Transaminaseinhibitors 3 zur spezifischen Hemmung der Transaminase TAM1 zur Prophylaxe und Therapie der Pityriasis versicolor wird in der WO 2007/054833 A2 nicht gezeigt.

Es ist im Stand der Technik kein Mittel bekannt, dass die Kleienflechte wirkungsvoll behandelt und zur Prophylaxe geeignet ist. Es besteht daher der Bedarf nach einem wirkungsvollen Mittel zur Behandlung und Prophylaxe der Kleienflechte.

Denn obwohl der Pilz durch die Antimykotika abgetötet wird, kommt es zum häufigen Wiederauftreten der Erkrankung. Wenn schon Depigmentierungen aufgetreten sind, führt zudem selbst eine Therapie mit Antimykotika nicht mehr zur Abheilung. Die Verwendung von Antimykotika insbesondere zur Prophylaxe ist sehr ungeeignet, da es zu einem großen Eintrag dieser Verbindungen in die Umwelt kommt, so daß auch Resistenzen bei anderen Pilzen induziert werden können. Ferner wird durch Einsatz der Antimykotika die Hautflora gestört, da Malassezia Hefen auch zur residenten Flora des Menschen gehören.

### Aufgabe

Aufgabe der vorliegenden Erfindung ist es, die Nachteile im Stand der Technik zu beseitigen und ein wirkungsvolles Mittel zur Behandlung und Prophylaxe der Kleienflechte bereitzustellen, das ein häufiges Wiederauftreten der Erkrankung verhindert und die Hautflora des Menschen schont.

### Lösung der Aufgabe

Die Aufgabe wird gelöst durch die Verwendung von mindestens einem Transaminaseinhibitor und einem Mittel bestehend aus mindestens einem Transaminaseinhibitor gemäß der Ansprüche. Der Transaminaseinhibitor bewirkt die Hemmung des für die Ausbildung der Erkrankung pathogenetisch bedeutsamen Transaminierungsprozesses wirkungsvoll und ist zur Behandlung und Prophylaxe der Kleienflechte geeignet, wobei ein Wiederauftreten der Erkrankung verhindert und die Hautflora des Menschen geschont wird.

Das erfindungsgemäße Mittel hat die Vorteile, dass es gut verträglich ist und besonders gut im Rahmen der Prophylaxe gegen Kleienflechte geeignet ist. Bei der Anwendung am Menschen wird die Hautflora geschont und beim Eintrag in die Umwelt werden keine Resistenzen bei anderen Pilzen induziert.
Das erfindungsgemäße Mittel ist auch besonders gut im Rahmen der Therapie gegen Kleienflechte geeignet, da der, der Erkrankung zu Grunde liegende Stoffwechselweg spezifisch durch mindestens einen erfindungsgemäßen Transaminaseinhibitor gehemmt wird.

Überraschenderweise wurde gefunden, dass das krankheitsauslösende Ereignis der Kleienflechte die Bildung von Pigmenten und Fluorochromen durch Malassezia-Hefen ist.

So ist beispielsweise *M*. *furfur* in der Lage, eine Reihe komplexer Indolverbindungen zu synthetisieren, wenn Tryptophan als alleinige Stickstoffquelle angeboten wird. Diese weisen interessante biologische Wirkungen auf und erklären die Pathogenese der Pityriasis versicolor.
So bewirkt Malassezin, der erste offenkettige Agonist des Aryl Hydrocarbon Rezeptors (AhR), eine dosis-abhängige Apoptose in menschlichen Melanozyten und trägt auf diese Weise zur Depigmentierung bei. Die UV-protektiven Eigenschaften des Pityriacitrins begründen, warum in den depigmentierten Arealen keine erhöhte UV-Empfindlichkeit auftritt. Die Pityriarubine A, B und C als Hemmstoffe des granulozytären Bursts stellen eine Erklärung für die geringe entzündliche Aktivität in den Läsionen trotz hoher Pilzlast dar. Das Fluorochrom Pityrialacton könnte die diagnostisch nutzbare Fluoreszenz der Läsionen erklären.
Weitere Untersuchungen an dem verwandten Organismus *Ustilago maydis* haben gezeigt, dass zur Biosynthese dieser enormen Vielzahl der komplexen Indolderivate aus Tryptophan nur ein einziger enzymatisch katalysierter Schritt notwendig ist. Dieser Schritt, die Desaminierung von Tryptophan zu Indolpyruvat, wird in *U*. *maydis* von der Tryptophan-Aminotransferase Tam1 katalysiert. Ein ähnliches Enzym wurde auch in Malassezia-Hefen gefunden.

Durch Einsatz eines Tryptophan-Aminotransferase Inhibitors wird die Pigmentbildung von *M. furfur* unterbunden. Dies zeigt, dass die Tam1 aus *M*. *furfur* für die tryptophanabhängige Pigmentbildung verantwortlich ist.

Die wissenschaftlichen Ergebnisse zeigen, dass in der Pathogenese der Pityriasis versicolor von Malassezia-Hefen synthetisierte, Tryptophan-abhängige Indolverbindungen bedeutend sind, die unter bestimmten Bedingungen, nämlich dem Fehlen oder Verbrauch anderer verwertbarer Aminostickstoffverbindungen im Hautmilieu, induziert werden. Sie entstehen nach Transaminierung des Tryptophans zu Indolpyruvat spontan aus diesem sowie in Reaktion mit verbleibendem Tryptophan.
Zur Biosynthese dieser enormen Vielzahl der komplexen Indolderivate aus Tryptophan ist nur ein einziger enzymatisch katalysierter Schritt notwendig, der von der Transaminase 1 (TAM 1) gesteuert wird.
Überraschenderweise konnte festgestellt werden, daß dieser für die Kleienflechte Pityriasis versicolor bedeutsame Tryptophan abhängige Stoffwechselweg durch den Einsatz des Transaminaseninhibitors Aminooxyacetat [*O-*(Carboxymethyl)hydroxylamine hemihydrochlorid; Molekularformel C2H5NO3.1/2HCl Molekulargewicht 109.29, CAS: 2921-14-4] vollständig gehemmt wird.
In einem Agardiffusionstest wird gezeigt, dass die Pigmentbildung völlig unterdrückt, das Wachstum des Pilzes aber nicht eingeschränkt ist.

Eine vergleichbare Unterdrückung der Pigmentbildung in vitro und in vivo am Menschen, wird auch beim Einsatz der Transaminaseinhibitoren D- Cycloserin (CAS: 68-41-7), DL-Cycloserin (CAS: 68-39-3) oder L-Cycloserin (CAS: 339-72-0) erzielt. Dies gilt auch für den Einsatz von Terizidon (CAS:25683-71-0), eines Derivates des D, DL oder L-Cycloserins, das ebenfalls die Pigmentbildung unterdrückt und daher als Mittel zur Prophylaxe und Therapie der Kleienflechte Pityriasis versicolor verwendet wird.

Damit stellt sich erfindungsgemäß ein neuer Ansatz zur Prophylaxe und Therapie der Kleienflechte Pityriasis versicolor besonders am Menschen dar. Er zielt vorrangig nicht auf antimykotische Effekte, die zu einer Abtötung des Pilzes führen, sondern auf eine Hemmung des für die Ausbildung der Erkrankung pathogenetisch bedeutsamen Transaminierungsprozesses. Damit wird die der Erkrankung zugrunde liegende Bildung von Tryptophan-abhängigen Pigmenten und somit die Ausprägung der Erkrankung überhaupt verhindert. Die Pityriasis versicolor beruht nach eigenen Ergebnissen auf einer Stoffwechselanpassung des Pilzes an veränderte Lebensbedingungen auf der Hautoberfläche und stellt im engeren Sinne keine Mykose/Infektion dar. Nach Aufbrauchen leicht verfügbarer Stickstoffquellen wie Glycin wird die Aminogruppe des Tryptophan auf Phenylpyruvat transaminiert. Das aus Trp entstandenen Indolpyruvat reagiert mit sich selbst und Tryptophan zu den Verbindungen, die die Pathogenese der Pityriasis versicolor bedingen. Spezifischer als der Einsatz von Antimykotika ist daher die Verwendung der erfindungsgemäßen spezifischen Inhibitoren zur Hemmung der Transaminase TAM1. Erfindungsgemäß werden die spezifischen Inhibitoren zur Hemmung der Transaminase TAM1 in topischer Anwendung zur Behandlung der Kleienflechte Pityriasis versicolor und auch zur Prophylaxe bereitgestellt. Besonders bevorzugt wird der Transaminaseninhibitor Aminooxyacetat eingesetzt, welcher im Agardiffusionstest ungepuffert oder gepuffert (pH 5,0) eine Wirksamkeit in einem Bereich von 0,05 bis > 0,5 Molar zeigt.
Alternativ werden als Transaminaseinhibitor auch Cycloserin wie D-Cycloserin (CAS: 68-41-7), DL-Cycloserin (CAS: 68-39-3) oder L-Cycloserin (CAS: 339-72-0) alleine oder in Kombination eingesetzt.

Alternativ wird als Transaminaseinhibitor auch das Cycloserin-Derivat Terizidon (CAS:25683-71-0) alleine oder in Kombination mit Stoffen ausgewählt aus der Gruppe Canalin (CAS: 39665-21-9), Tunicamycin (CAS: 11089-65-9), GABA (CAS:56-12-2), Hydrazine hydrochlorid (CAS: 304-20-1), S(+)-y-Vigabatrin (CAS: 74046-07-4), Gabaculin hydrochlorid (CAS: 59556-17-1), Ibotenic acid (CAS:2552-55-8), γ-Guanidinobuttersäure (CAS: 463-00-3) eingesetzt.
Die Hemmung der TAM1 erfolgt dabei kompetitiv oder nicht-kompetitiv.

Erfindungsgemäß wird der mindestens eine Transaminaseninhibitor in topischer Anwendung beispielsweise als Shampoo, Lösung, Lotion, Creme und Salbe eingesetzt. Alternativ werden die Transaminaseninhibitoren in Kombination mit Juckreiz-stillenden Mitteln, insbesondere Polidocanol (CAS: 3055-99-0) eingesetzt. Polidocanol wirkt zudem in 5-10%iger Konzentration antimykotisch gegenüber Malassezia-Hefen. Da die Transaminierung von Tryptophan erst bei einem Mangel an weiteren verwertbaren Stickstoffquellen im Hautmilieu induziert wird, ist in der Anwendungsform auch die Kombination dieser für Malassezia-Hefen verwertbaren Stickstoffquellen umfasst. Beispielsweise gehört zu solchen Stickstoffquellen Urea (Harnstoff), welcher von Malassezia-Hefen verwertet wird und gleichzeitig einen pflegenden und feuchtigkeitsstabilisierenden Effekt auf die Haut entfaltet. Alternativ wird der mindestens eine Transaminaseninhibitor in Kombination mit bekannten Antimykotika eingesetzt. Weiterhin ist die Kombination mit anderen dem Fachmann bekannten und geläufigen Verbindungen möglich. Erfindungsgemäß wird das Mittel zur Behandlung und Prophylaxe der Kleienflechte insbesondere der spezifische Inhibitor zur Hemmung der Transaminase TAM1 in topischer Anwendung beispielsweise als Shampoo, Lösung, Lotion, Creme, Salbe in Kombination mit Polidocanol oder Antimykotika oder anderen geeigneten Verbindungen, die der Fachmann kennt, eingesetzt.

Nachfolgend wird die Erfindung anhand von Beispielen näher erläuert.

### Ausführungsbeispiele

### 1. Induktion des Pigmentweges

Als eine Besonderheit des Stickstoffstoffwechsels von *Malassezia-Hefen* zeigt sich eine Synthese von Pigmenten und Fluorochromen, wenn Tryptophan (TRP) als alleinige Stickstoffquelle angeboten wird. Dieser Effekt lässt sich insbesondere bei der Spezies *M. furfur* induzieren und ist zudem bei einem Teil von *M. pachy*dermatis-Isolaten nachweisbar.

Das Pigmentinduktionsmedium (P-Agar) besteht aus (für 1 Liter Medium)

| | | |
|---|---|---|
| 20 g | Agar | z.B. Merck, Darmstadt, BRD |
| 990 ml | Aqua dest. | z.B. Pharmacia, Erlangen, BRD |

Nach 30 min Autoklavieren bei 1 bar wird dazu gegeben:

| | | |
|---|---|---|
| 30 ml | Tween 80 | z.B. Sigma, Deishofen, BRD |

Bei ca. 50 °C wegen Hitzelabilität wird zugegeben:

| | | |
|---|---|---|
| 10g | L-Tryptophan | z.B. Sigma |

Der pH-Wert liegt bei pH 5,0.
Jeweils 25 ml des Mediums werden in Petrischalen von 10 cm Durchmesser ausgegossen.
Eine Suspension von M. *furfur* CBS 1878 (etwa 5 Ösen eines zuvor auf Dixon-Agar angezüchteten Stammes) werden in 1 ml Aq. dest. suspendiert, auf den P-Agar aufgegeben und durch Schwenken verteilt. Bereits nach 1 tägiger Inkubation bei 32°C in einem Brutschrank wird eine Braunverfärbung des Mediums als Ausdruck der Pigmentbildung sichtbar und ist optisch nachweisbar. Die Zusammensetzung des Pigmentes lässt sich nach Extraktion und dünnschichtchromatographischer Auftrennung darstellen.

### 2. Hemmung des Pigmentweges durch mindestens einen Transaminaseinhibitor

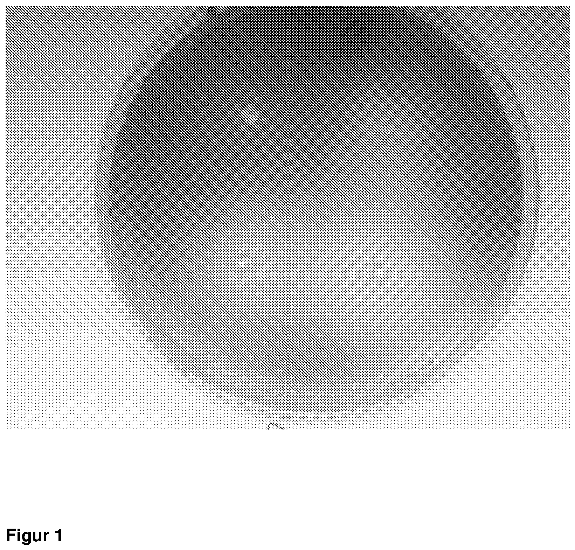

Die komplette Hemmung der Pigmentsynthese wird beispielsweise im Agardiffusionstest dargestellt. Hierzu wird M. *furfur* CBS 1878 wie oben beschrieben auf einer Agarplatte mit Pigmentinduktionsmedium verteilt und anschließend mit einer sterilen Stanze 4mm große Löcher angebracht, in die jeweils 20µl Transaminaseinhibitor Aminooxyacetatlösung (z.B. Sigma) oder Cycloserin z.B. D- Cycloserin, DL-Cycloserin, L-Cycloserin oder Terizidon in verschiedenen Konzentrationen eingebracht wird. Das Ergebnis mit Aminooxyacetatlösung und einer 4 Tage alten Kultur von M. *furfur* CBS 7019 auf pigment-induzierendem Medium mit Tryptophan (Trp) als alleiniger Stickstoffquelle zeigt Figur 1. Der Agardiffusionstest wird mit Transaminaseinhibitor z.B. Aminooxyacetat (Sigma) in Wasser, gepuffert mit 1n NaOH auf pH 5 durchgeführt. Die Konzentrationen betragen links oben 0,05; rechts oben 0,1; links unten 0,25 und rechts unten 0,5 molar. Wie in Figur 1 zu sehen ist, bleibt durch den Einsatz des Transaminaseinhibitor z.B. Aminooxyacetat eine Pigmentbildung aus, ferner wird das Wachstum des Pilzes gehemmt, da in der speziellen Situation Stickstoff durch die unterbundene Transaminierung nicht verfügbar werden kann und andere Stickstoffquellen nicht mehr zur Verfügung stehen.

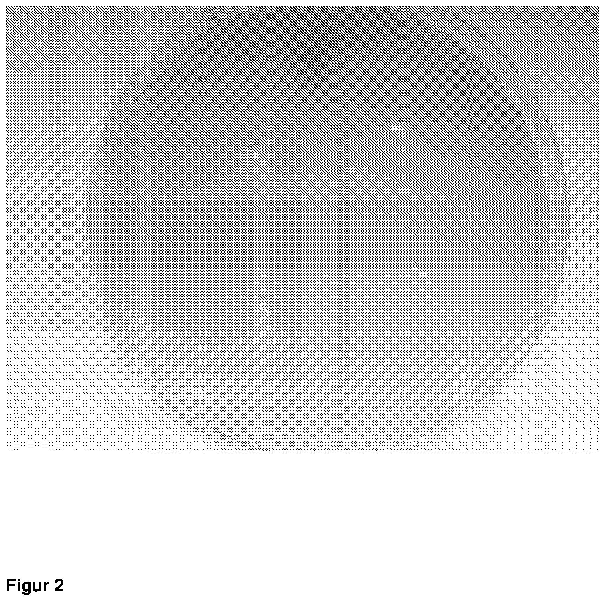

Figur 2 zeigt, dass die Hemmwirkung der Pigmentbildung und damit auch ein ausbleibendes Wachstum des Pilzes nicht auf einem antimykotischen Effekt des mindestens einen Transaminaseinhibitors, sondern auf einer spezifischen Hemmung der Transaminase beruht. Dazu wird der identische Versuchsansatz wie oben beschrieben gewählt und der Pilz auf einem Komplettmedium Dixon-Agar für 4 Tage angezogen. Transaminaseinhibitor Aminooxyacetatlösung (z.B. Sigma) oder D-Cycloserin, DL-Cycloserin, L-Cycloserin oder Terizidon wird in den genannten Konzentrationen zu Versuchsbeginn eingebracht. Eine Pigmentbildung wird nicht induziert, da mehrere Stickstoffquellen zur Verfügung stehen. Eine spezifische Hemmung der Transaminierung durch Aminooxyacetat in Konzentration wie unter Figur 1 führt zu keinen Effekten, da die Hemmung umgangen werden kann.

Auch ein toxischer Effekt ist nicht nachweisbar. Das Pilzwachstum ist gleichmäßig, auch im Bereich der Diffusionshöfe. Die in Abb. 1 beobachtbare Hemmung beruht daher auf einem spezifischen Effekt.

### 3. Anwendung am Menschen

Die Transaminaseninhibitoren Aminooxyacetat, Cycloserin z.B. D- Cycloserin, DL-Cycloserin, L-Cycloserin oder Terizidon sind zum Einsatz beim Menschen geeignet. Eine topische Anwendung von 5% in Unguentum emulsificans aquosum, angewendet über mehrere Tage, wird problemlos ohne Nebenwirkungen vertragen. Für eine prophylaktische Anwendung wird eine 0,05 - 5% w/w Zubereitung bevorzugt als Schüttelmixtur/Schüttelpinselung, wässrige, ölige oder alkoholische Suspension, Emulsion, Paste, Creme, Salbe, Spray, lipophiles Gel, hydrophiles Gel oder Pflaster, Shampoo, Badezusatz oder Schlamm eingesetzt, da nach Möglichkeit der gesamte Körper behandelt werden sollte. Die Anwendung erfolgt prophylaktisch täglich bis hin zu mehrtägigen Abständen. Gleiches gilt für die therapeutische Anwendung.

D-Cycloserin wird bevorzugt in einer wässrigen Lösung z.B. 0,2 mol, gepuffert auf pH 7 z.B. mit Phosphatpuffer verwendet. Diese Lösung zeigte bei der anwendung am Menschen bei 2 mal täglicher Anwendung schon nach 3 Tagen eine völlige Abheilung der Läsionen, bei sehr guter Verträglichkeit ohne Nebenwirkungen.

### 4. Formulierung

Das erfindungsgemäße, mindestens einen Transaminaseninhibitor enthaltende Mittel zur Behandlung und Prophylaxe der Kleienflechte wird in jeder dem Fachmann bekannten Art und Weise hergestellt und formuliert. Für die topische Applikation ist geeignet z.B. Schüttelmixtur/Schüttelpinselung, wässrige, ölige oder alkoholische Suspension, Emulsion, Paste, Creme, Salbe, Spray, lipophiles Gel, hydrophiles Gel oder Pflaster, auch die Formulierung als medizinisches Shampoo, Badezusatz oder Schlamm ist geeignet.

Für die spezielle Herstellung kennt der Fachmann Verfahren aus dem Stand der Technik und stellt beispielsweise Öl- und Feuchtigkeitsgehalt einer Creme, sowie pH-Wert ein. Shampoo, Lösung, Gel, Creme und Salbe enthält alternativ zusätzliche Vitamine, UV-Schutz und Duftstoffe.

Weitere Bestandteile die bei der Formulierung der erfindungsgemäßen Zusammensetzungen verwendet werden, umfassen z.B. (thixotrope) hydrophile Konsistenzgeber wie Methyl-, Hydroxypropylcellulose, Xanthangummi, Polyacrylsäuren, Stärke, Gelatine, Alginate, Kieselsäure, Magnesiumaluminiumsilikat, Bentonit; (thixotrope) lipophile Konsistenzgeber wie Wollwachs, Glycerolmonostearat, Cetylpalmitat, Cetylstearylalkohol, Bienenwachs und Hartparaffin; Verdünnungsmittel bzw. Dispersionsmittel wie Wasser, einwertige Alkohole wie Ethanol, Isopropanol oder deren Mischungen und mehrwertige Alkohole wie Glycole; fette Öle wie Sojaöl, Kokosnussöl, Erdnussöl, Avocadoöl, Nachtkerzenöl, Olivenöl, Baumwollsaatöl, Saffloröl; Ethyloleat, Isopropylmyristat und Oleyloleat; Siliconöle wie Dimethicon; und flüssige oder halbfeste Kohlenwasserstoffe wie Vaseline; Hartparaffin, Ceresin, Mineralöle bzw. Paraffinöle oder niedrigschmelzende Wachse, Solubilisierungsmittel; Stabilisierungsmittel; Puffer, Emulgatoren und Tenside, wie Fettsäureester wie z.B. Laureth-4, Steareth-2, Ceteareth-12, Oleth-5 oder Sorbitanfettsäureester wie z.B. Sorbitanmonolaurat, -stearat, oder Polysorbat 20, 40 oder 60; Polyglycerylester, Zuckerester oder Rizinusölverbindungen; Benetzungsmittel, Feuchthaltemittel wie Glycerin, Propylenglycol, Hexylenglycol, Butandiol und Sorbitol; Fettsäuren/Fettalkohole; Farbstoffe, Konservierungsmittel wie Benzoe-, Sorbinsäure, Pheoxyethanol, Benzylalkohol, Parabene und Benzalkoniumchlorid; und Antioxidantien wie alphatocopherol. Ascorbinsäure, Butylhydroxytoloul, Propyl-, Dodecylgallat sowie Synergisten wie Citronensäure oder EDTA; Feststoffe wie Talkum, Titan(IV)oxid, Mais-, Reisstärke oder hochdisperses Siliciumdioxid; Neutralisationsmittel wie Natronlauge, Triethanolamin oder Trometamol; Filmbildner wie Polyvidon oder PVP-Copolymere und Penetrationsförderer wie Diethylenglycolmonoethylether.

Zudem enthält das erfindungsgemäße, mindestens einen Transaminaseinhibitor enthaltende Mittel zur Behandlung und Prophylaxe der Kleienflechte feuchtigkeitsbindende Stoffe wie Harnstoff. Auch hydrophile Grundlagen z.B. mit Carboxymethylcellulose als Gelbildner werden erfindungsgemäß verwendet. Für Suspensionen und Schüttelmixturen/-pinselungen sind Wasser, Mischungen aus AIkohol/Wasser, aus Wasser und Ethanol oder Isopropanol als Dispersionsmittel geeignet.

Als Grundlagen für ölige Suspensionen und Schüttelmixturen werden insbesondere fette Öle wie Olivenöl, Erdnussöl, Nachtkerzenöl oder Kokosnussöl verwendet. Als Grundlage für Pasten eignet sich insbesondere auch Pflanzen(Birken)teer. Der Gehalt der topischen Zusammensetzungen an Transaminaseinhibitor Aminooxyacetat oder D- Cycloserin, DL-Cycloserin, L-Cycloserin oder Terizidon beträgt etwa 0,01 % bis 99 %, vorzugsweise 0,05 % bis 50 %, insbesondere bevorzugt ist ein Gehalt von 0,5 - 5%.
In einer bevorzugten Ausführungsform wird D-Cycloserin (CAS: 68-41-7, 204,2 mg) in Kaliumdihydrogenphosphat/Kaliumhydrogenphosphat Puffer verwendet, z. B. als 10 ml Lösung, so dass D-Cycloserin (CAS: 68-41-7, 204,2 mg) in einem wässrigen Kaliumdihydrogenphosphat (5 mg)/Kaliumhydrogenphosphat (167 mg)-Puffer, bei pH 7 vorliegt.

## Patentansprüche

1. Verwendung mindestens eines Transaminaseinhibitors zur Herstellung eines Mittels zur Prophylaxe und Therapie der Pityriasis versicolor (Kleienflechte) **dadurch gekennzeichnet, dass** der Transaminaseinhibitor Aminooxyacetat, Cycloserin oder das Derivat von Cycloserin, nämlich Terizidon, ist.

2. Verwendung nach Anspruch 1 **dadurch gekennzeichnet dass** der Transaminaseinhibitor spezifisch die Transaminase TAM1 hemmt.

3. Verwendung nach Anspruch 1 und 2 **dadurch gekennzeichnet, dass** der Transaminaseinhibitor Terizidon ist.

4. Verwendung gemäß der vorangegangenen Ansprüche in einer Schüttelmixtur, Schüttelpinselung, wässrigen, öligen oder alkoholischen Suspension, als Emulsion, Paste, Creme, Salbe, Spray, lipophiles Gel, hydrophiles Gel, Pflaster, Shampoo, Badezusatz oder Schlamm.

5. Verwendung gemäß der vorangegangenen Ansprüche, **dadurch gekennzeichnet dass** der Transaminaseinhibitor in einer Konzentration von 0,05 - 5 % w/w enthalten ist.

6. Mittel zur Verwendung bei der Prophylaxe und Therapie der Pityriasis versicolor (Kleienflechte) **dadurch gekennzeichnet, dass** es mindestens einen Transaminaseinhibitor umfasst, der Aminooxyacetat, Cycloserin oder das Derivat von Cycloserin, nämlich Terizidon, ist.

7. Mittel zur Verwendung gemäß Anspruch 6 **dadurch gekennzeichnet, dass** es spezifisch die Transaminase TAM1 hemmt.

8. Mittel zur Verwendung gemäß Anspruch 6 **dadurch gekennzeichnet, dass** der Transaminaseinhibitor Terizidon ist.

9. Mittel zur Verwendung gemäß Anspruch 6 bis 8 **dadurch gekennzeichnet, dass** es als Schüttelmixtur, Schüttelpinselung, wässrige, ölige oder alkoholische Suspension, Emulsion, Paste, Creme, Salbe, Spray, lipophiles Gel, hydrophiles Gel oder Pflaster, Shampoo, Badezusatz oder Schlamm vorliegt.

10. Mittel zur Verwendung gemäß Anspruch 6 bis 9 **dadurch gekennzeichnet, dass** es eine juckreizstillende Substanz umfasst.

11. Mittel zur Verwendung gemäß Anspruch 6 bis 10 **dadurch gekennzeichnet, dass** es (Amino-) Stickstoffhaltigen Verbindungen umfasst.

12. Mittel zur Verwendung gemäß Anspruch 6 bis 11 **dadurch gekennzeichnet, dass** der Transaminaseinhibitor in einer Konzentration von 0,05 - 5 % enthalten ist.

## Claims

1. Use of at least one transaminase inhibitor for the production of an agent for preventing and treating pityriasis versicolor (tinea versicolor), **characterised in that** the transaminase inhibitor is aminooxyacetate, cycloserine or the derivative of cycloserine, namely terizidone.

2. Use according to claim 1, **characterised in that** the transaminase inhibitor specifically inhibits the transaminase TAM1.

3. Use according to claims 1 and 2, **characterised in that** the transaminase inhibitor is terizidone.

4. Use according to the preceding claims in a shaking mixture, brush-on shaking preparation, aqueous, oily or alcoholic suspension, as emulsion, paste, cream, ointment, spray, lipophilic gel, hydrophilic gel, patch, shampoo, bath additive or mud.

5. Use according to the preceding claims, **characterised in that** the transaminase inhibitor is contained in a concentration of 0.05 - 5% w/w.

6. Agent for use in the prevention and treatment of pityriasis versicolor (tinea versicolor), **characterised in that** it comprises at least one transaminase inhibitor that is aminooxyacetate, cycloserine or the derivative of cycloserine, namely terizidone.

7. Agent for use according to claim 6, **characterised in that** it specifically inhibits the transaminase TAM1.

8. Agent for use according to claim 6, **characterised in that** the transaminase inhibitor is terizidone.

9. Agent for use according to claims 6 to 8, **characterised in that** it exists as shaking mixture, brush-on shaking preparation, aqueous, oily or alcoholic suspension, emulsion, paste, cream, ointment, spray, lipophilic gel, hydrophilic gel or patch, shampoo, bath additive or mud.

10. Agent for use according to claims 6 to 9, **characterised in that** it comprises an antipruritic substance.

11. Agent for use according to claims 6 to 10, **characterised in that** it comprises (amino-) nitrogen-containing compounds.

12. Agent for use according to claims 6 to 11, **characterised in that** the transaminase inhibitor is contained in a concentration of 0.05 - 5%.

## Revendications

1. Utilisation d'au moins un inhibiteur de transaminase pour la fabrication d'un agent destiné à la prévention et au traitement du Pityriasis versicolor, **caractérisée en ce que** l'inhibiteur de transaminase est l'aminooxyacétate, la cyclosérine ou le dérivé de la cyclosérine, à savoir la térizidone.

2. Utilisation selon la revendication 1, **caractérisée en ce que** l'inhibiteur de transaminase inhibe spécifiquement la transaminase TAM1.

3. Utilisation selon les revendications 1 et 2, **caractérisée en ce que** l'inhibiteur de transaminase est la térizidone.

4. Utilisation selon les revendications précédentes dans une mixture à agiter, dans une mixture à agiter et à badigeonner, dans une suspension aqueuse, huileuse ou alcoolique, sous forme d'émulsion, de pâte, de crème, de pommade, de spray, de gel lipophile, d'emplâtre, de shampooing, d'adjuvant pour le bain, de boue.

5. Utilisation selon les revendications précédentes, **caractérisée en ce que** l'inhibiteur de transaminase est contenu avec une concentration de 0,05 à 5 % masse/masse.

6. Agent pour une utilisation lors de la prévention et du traitement du Pityriasis versicolor, **caractérisé en ce qu'**il comprend au moins un inhibiteur de transaminase qui est l'aminooxyacétate, la cyclosérine ou le dérivé de la cyclosérine, à savoir la térizidone.

7. Agent pour une utilisation selon la revendication 6, **caractérisé en ce qu'**il inhibe spécifiquement la transaminase TAM1.

8. Agent pour une utilisation selon la revendication 6, **caractérisé en ce que** l'inhibiteur de transaminase est la térizidone.

9. Agent pour une utilisation selon les revendications 6 à 8, **caractérisé en ce qu'**il se présente sous la forme d'une mixture à agiter, d'une mixture à agiter et à badigeonner, d'une suspension aqueuse, huileuse ou alcoolique, d'une émulsion, d'une pâte, d'une crème, d'une pommade, d'un spray, d'un gel lipophile, d'un emplâtre, d'un shampooing, d'un adjuvant pour le bain, d'une boue.

10. Agent pour une utilisation selon les revendications 6 à 9, **caractérisé en ce qu'**il comprend une substance calmant le prurit.

11. Agent pour une utilisation selon les revendications 6 à 10, **caractérisé en ce qu'**il comprend des composés (amino-) azotés.

12. Agent pour une utilisation selon les revendications 6 à 11, **caractérisé en ce que** l'inhibiteur de transaminase est contenu avec une concentration de 0,05 à 5 % masse/masse.
